# EUROPEAN PATENT APPLICATION

(11) **EP 4 571 313 A1**
(43) Date of publication of application: **18.06.2025**
(21) Application number: 23844522.5
(22) Date of filing: 30.11.2023
(51) Int. Cl.: G01N 33/573, G01N 33/58

(54) **METHOD FOR DIAGNOSING ACUTE EXACERBATION OF BRONCHIECTASIS AND KIT THEREFOR**

(30) Priority: 26.10.2023 KR 20230145128
(71) Applicant: Z Biotech Inc., Cheongju-si, Chungcheongbuk-do 28576 (KR)
(72) Inventor: The designation of the inventor has not yet been filed
(74) Representative: Herrero & Asociados, S.L.
(86) International application number: PCT/KR2023/019612
(87) International publication number: WO 2025/089489

(57) **Abstract**

The present disclosure relates to a method for predicting acute exacerbation of bronchiectasis. The use of the method and kit of the present disclosure allows for easy diagnosis of acute exacerbation of bronchiectasis through a quantitative indicator.

## Description

### Technical Field

This application claims priority and the benefit of Korean Patent Application No. 10-2023-0145128 filed in the Korean Intellectual Property Office on 26 October 2023, the disclosure of which is incorporated herein by reference.

The present disclosure relates to a method for predicting acute exacerbation of bronchiectasis and a kit therefor.

### Background Art

Bronchiectasis is a condition where the bronchi are permanently enlarged to the extent that they cannot return to their original state. These symptom sites may be partially shown or extensively shown throughout the entire lung. The affected airway has various changes, such as inflammation, mucosal swelling, ulcers, neovascularization of bronchial arteries, scarring, or bronchus twisting due to obstruction caused by recurrent infections. Bronchial obstruction by lung secretions may cause pneumonia, which may also cause parenchyma damage temporarily or permanently. Factors resulting in bronchiectasis are as follows: 1) Infectious damage, 2) airway obstruction, 3) impaired clearance of mucus and airway secretions, and 4) impairment of host defense mechanism.

Meanwhile, bronchiectasis occurs due to an inflammatory response and destruction of the elements constituting the bronchial wall. The most common cause of an inflammatory response is an infection, and some microorganisms secrete pigments, proteolytic enzymes, and toxins that damage the respiratory epithelium and impair the ability of cilia to clear mucus. An inflammatory response in a host damages the epithelium mainly through media secreted from neutrophils, and as the resistance to infection decreases, bacterial colonization or growth occurs in the enlarged bronchi. The inflammatory response causes airway damage, reduced ability to clear microorganisms, and additional infections, resulting in a more severe inflammatory response.

Methods for diagnosing bronchiectasis include physical examinations, such as asking conditions, evaluating breathing sound changes and dyspnea state through chest auscultation, simple chest radiography and chest high-resolution computer tomography, sputum bacterial culture testing, bronchoscopy, bronchography, and lung function testing. However, no screening method available for objective and quantitative detection of bronchiectasis.

Bronchiectasis has a wide variety of clinical features, ranging from patients with few respiratory symptoms to patients requiring lung transplantation due to severe structural lung damage with frequent acute exacerbations. Consequently, patients may have various prognoses. Recent research results show that the prevalence of such bronchiectasis is increasing, and the rate of hospitalization related to the disease is increasing; thus bronchiectasis is considered a disease with a high burden of disease. Therefore, an objective and useful severity classification system can accurately reflect the prognosis of the disease to treat and manage bronchiectasis patients appropriately. In recent years, several indicators have been developed that can effectively assess the severity of bronchiectasis in consideration of several clinical factors and radiological findings.

Apart from the classification of bronchiectasis severity, acute exacerbation may occur in all patients ranging from mild to severe. The acute exacerbation that may occur in bronchiectasis patients may include a higher sputum volume than usual, exacerbated purulence, blood in the sputum, and significant systemic symptoms including fever. However, it cannot be easily determined whether the manifestation of the symptoms mentioned above is due to acute exacerbations, and no diagnostic kit has been developed to determine these symptoms in the previous stage or initial stage of acute exacerbations.

Many patent documents are referenced throughout the specification and their citations are represented. The disclosures of cited papers and patent documents are entirely incorporated by reference herein, and the technical field level within which the present disclosure falls and details of the present disclosure are explained more clearly.

### Detailed Description of the Invention

### Technical Problem

The present inventors have made intensive research efforts to develop a method and diagnostic kit capable of diagnosing acute exacerbation of bronchiectasis by an objective and quantitative indicator. As a result, the present inventors confirmed that patients in a clinical state of acute exacerbation (AE) of bronchiectasis commonly show a sputum MPO concentration exceeding a predetermined level and established that the corresponding indicator can objectively and quantitatively identify acute exacerbation of bronchiectasis, and thus completed the present disclosure.

Accordingly, an aspect of the present disclosure is to provide an information providing method for diagnosing acute exacerbation of bronchiectasis.

Another aspect of the present disclosure is to provide a diagnostic kit for diagnosing acute exacerbation of bronchiectasis.

### Technical Solution

In accordance with an aspect of the present disclosure, there is provided an information-providing method for diagnosing acute exacerbation of bronchiectasis, the method including measuring the myeloperoxidase (MPO) concentration in a biological sample isolated from a subject, wherein the subject is determined as being in an acute exacerbation state of bronchiectasis if the measured MPO concentration exceeds 50 ng/mL.

The present inventors have made intensive research efforts to develop a method and diagnostic kit capable of diagnosing acute exacerbation of bronchiectasis by an objective and quantitative indicator. As a result, the present inventors confirmed that patients in a clinical state of acute exacerbation (AE) of bronchiectasis commonly show a sputum MPO concentration exceeding a predetermined level, and established that the corresponding indicator can objectively and quantitatively identify acute exacerbation of bronchiectasis.

The term "subject," as used herein, refers to a specimen suspected of an acute exacerbation state of bronchiectasis. The subject may be a mammal that may develop bronchiectasis and more specifically, the subject includes a human patient suspected of developing bronchiectasis or diagnosed with developing bronchiectasis.

In an embodiment of the present disclosure, the "subject" is a subject suffering from bronchiectasis. Since the information-providing method of the present disclosure can be preferably used to determine whether a subject with bronchiectasis is in an acute exacerbation state of bronchiectasis, the above-described "subject" may be effectively used for a patient diagnosed with bronchiectasis.

In an embodiment of the present disclosure, the "subject" is a subject without an acute exacerbation state within three weeks. In the information-providing method of the present disclosure, if the subject suffers from an acute exacerbation state within three weeks from the performing of the step of measuring the myeloperoxidase (MPO) concentration in a biological sample isolated from the subject, the standard of the MPO concentration for determining whether the subject has been stabilized from an acute exacerbation state to a stable state may be a value higher than 50 ng/mL.

The term "biological sample," as used herein, may be anyone selected from sputum, saliva, blood, serum, or alveolar lavage fluid, which are collected from the above-mentioned subject by various methods.

In an embodiment of the present disclosure, the "biological sample" is sputum. The acute exacerbation of bronchiectasis is usually accompanied by the production of sputum or an increased volume of sputum produced, in which case the subject may be suspected of an acute exacerbation state. For objective and quantitative identification of an acute exacerbation state, sputum may be collected from a patient and helpfully used as a biological sample according to an embodiment of the present disclosure.

The term "myeloperoxidase (MPO)" as used herein, also called myeloid peroxidase, is one of the most abundant proteins in neutrophils and one of the main enzymes secreted out of the cell as neutrophils are activated so that the myeloperoxidase (MPO) is used as an indicator of neutrophil activity. MPO can contribute to the migration of neutrophils to an inflammation site and catalyze the production of highly oxidative hypochlorous acid from hydrogen peroxide and chloride ions and thus may cause damage to the alveoli when excessive inflammation occurs in the lungs. It is known that the MPO concentration increases in inflammatory conditions or damaged lungs. However, very little is known about the correlation between the MPO concentration and the presence of acute exacerbation, regardless of the severity of bronchiectasis, that may occur in patients from the beginning of the disease onset to the progression to severe disease.

The MPO of the present disclosure may be a protein having the amino acid sequence set forth in SEQ ID NO: 1 or a protein having an amino acid sequence having a homology of at least 90%, at least 95%, or at least 98% to the amino acid sequence of SEQ ID NO: 1. SEQ ID NO: 1 may consist of the sequence corresponding to UNIPROT ID: P05164.

[SEQ ID NO: 1]















The term "acute exacerbation," as used herein, specifically refers to the acute exacerbation of bronchiectasis, and clinically acute exacerbation refers to the exacerbation of symptoms, such as coughing, changes in sputum, and dyspnea in a bronchiectasis patient. Through systematic paper review and discussion by several bronchiectasis experts, Hill et al. (Hill AT, Haworth CS, Aliberti S, Barker A, Blasi F, Boersma W, et al. Pulmonary exacerbation in adults with bronchiectasis: a consensus definition for clinical research. Eur Respir J 2017;49) defined the acute exacerbation of bronchiectasis for clinical research as follows. Acute exacerbation is a state where a bronchiectasis patient has a deterioration in three or more of the following features lasting for at least 48 h: (i) cough, (ii) changes in sputum volume and viscosity, (iii) sputum purulence, (iv) dyspnea and/or exercise tolerance; fatigue and/or malaise, wherein when a doctor decides to change the treatment of bronchiectasis, the acute exacerbation state of bronchiectasis of the present disclosure is expected to be determined as being a clinically acute exacerbation state based on the above criteria.

In an information-providing method, according to an embodiment of the present disclosure, the subject can be identified as being in an acute exacerbation state of bronchiectasis if the MPO concentration measured from the above-described biological sample exceeds 50 ng/mL. The present inventors have derived the fact that it could be objectively determined whether patients diagnosed with bronchiectasis according to the current clinical criteria are in an acute exacerbation state or a stable state by a quantitative indicator of the MPO concentration in a biological sample collected from the patients, and have established that the patients can be determined as being in an acute exacerbation state if the MPO concentration in the biological sample exceeds 50 ng/mL. The present inventors identified that the MPO concentration in the biological sample correlates with an acute exacerbation state of bronchiectasis and first presented an objective standard therefor. According to a specific embodiment of the present disclosure, the MPO concentration in a biological sample assessed as being in an acute exacerbation state may be defined as a value exceeding 50 ng/mL, but may be presented as a changed value by reflecting various characteristics of a patient group. Specifically, for example, the upper limit of MPO concentration in determining whether a patient group suffering from acute exacerbation in the near term, for example, within one, two, three, or four weeks, have recovered to a normal state, may be defined as a value higher than 50 ng/mL, for example, 60 ng/mL, 65 ng/mL, 70 ng/mL, 75 ng/mL, or 80 ng/mL. If the patients have an MPO concentration below the corresponding standard, the patients may be determined as being in a normal state.

In an embodiment of the present disclosure, the measuring of the myeloperoxidase (MPO) concentration may be performed by the following steps:
(a) mixing the biological sample with a detection buffer comprising a first anti-MOP antibody labeled with a fluorescent element;
(b) allowing the mixture in step (a) to react with a membrane coated with a second anti-MPO antibody; and
(c) measuring the MPO concentration in the biological sample through fluorescent detection of the fluorescent element after step (b).

The first anti-MPO antibody and the second anti-MPO antibody, according to the embodiment of the present disclosure, may be various antibodies capable of specifically binding to MPO without limitation. Preferably, antibodies that are newly designed and prepared based on the MPO protein sequence according to SEQ ID NO: 1, and existing commercially accessible anti-MPO antibodies, may be used.

In the above-described embodiment, the first anti-MPO antibody and the second anti-MPO antibody may be heterogeneous or homologous types, and the types thereof are not limited. Specifically anti-MPO antibodies that are commercially available at the time of completion of the present disclosure may be preferably used, and anti-MPO antibodies to be developed in the future may also be preferably used for the implementation of the present disclosure.

In an embodiment of the present disclosure, the following step may be further performed before step (a):
(pre-a) diluting the biological sample with a dilution buffer.

The biological sample can be further stabilized by dilution with a dilution buffer and, specifically, for example, when sputum is used as a biological sample, the accuracy in the MPO concentration measurement can be improved by diluting the sputum with a dilution buffer due to the characteristics of highly viscous sputum.

In an embodiment of the present disclosure, the biological sample may be diluted with the dilution buffer at a volume ratio of 1:5(v/v) to 1:20(v/v) in the above-described step (pre-a). More specifically, for example, the biological sample may be diluted with the dilution buffer at a volume ratio of 1:5 to 1:15(v/v), 1:8 to 1:12(v/v), or most specifically, 1:10(v/v), but is not limited thereto.

The detection buffer in step (a), according to an embodiment of the present disclosure, may be mixed with the above-described biological sample or a mixture of the above-described biological sample and dilution buffer at a volume ratio of 1:5(v/v) to 1:20(v/v). The above-described volume ratio corresponds to a range that is arbitrarily set for easy detection but is not necessarily limited thereto.

The term "fluorescent element," as used herein, is any fluorescent element that can be used for immunofluorescence analysis, wherein it would be evident that any known fluorescent element can be appropriately used and is not particularly limited.

In an embodiment of the present disclosure, the fluorescent element may be any one selected from the group consisting of Indocyanine green (ICG), IRDye 800CW carboxylate (IRDye 800CW), Alexa Fluor-355, Alexa Fluor 488, Alexa Fluor 532, Alexa Fluor 546, Alexa Fluor-555, Alexa Fluor 568, Alexa Fluor 594, Alexa Fluor 647, Alexa Fluor 660, Alexa Fluor 680, Alexa Fluor 700, Alexa Fluor 750, Alexa Fluor 780, Flamma 749, Flamma 774, Flamma 800, FSD FluorTM 647, FSD FluorTM 680, FSD FluorTM 750, FSD FluorTM 800, sulfo-cyanine 3.5 carboxylic acid (Cy3.5), sulfo-cyanine 5 carboxylic acid (Cy5), sulfo-cyanine 5.5 carboxylic acid (Cy5.5), sulfo-cyanine 7 carboxylic acid (Cy7) or sulfo-cyanine 7.5 carboxylic acid (Cy7.5), Texas Red, Pacific Blue, Oregon Green 488, JOE, Lissamine, Rhodamine Green, BODIPY, fluorescein isothiocyanate (FITC), carboxy-fluorescein (FAM), Allophycocyanin (APC), phycoerythrin (PE), rhodamine, dichlororhodamine (dRhodamine), carboxy tetramethylrhodamine (TAMRA), carboxy-X-rhodamine (ROX), PicoGreen, and RiboGreen.

In accordance with another aspect of the present disclosure, there is provided a kit for diagnosing acute exacerbation of bronchiectasis, the kit comprising a membrane coated with an anti-MPO antibody or an antigen-binding fragment thereof, wherein the kit is provided in the form of a cartridge for immunofluorescence measurement.

As a specific example, the presence of acute exacerbation of bronchiectasis can be determined by quantitatively analyzing the light emission by the fluorescent element bound to the detection antibody through sandwich immunoassay in which antibodies bind to an antigen therebetween.

In an embodiment of the present disclosure, the kit may include: a pad for a sample, on which a mixture of a biological sample and a detection antibody can be dropped; an absorption pad for sucking a solution to move the biological sample; and a test line, onto which an anti-MPO antibody is fixed. The kit may further include a control line capable of detecting a contrast protein.

In an embodiment of the present disclosure, the membrane of the kit may be a nitrocellulose membrane. The nitrocellulose membrane is a material suitable for coating and fixing an antibody and can be helpfully used in an embodiment of the present disclosure.

The "kit for diagnosing acute exacerbation of bronchiectasis" as an aspect of the present disclosure is a diagnostic kit for use in the implementation of the "information-providing method for diagnosing acute exacerbation of bronchiectasis" as another aspect of the present disclosure and thus recites the overlapping contents therebetween. The description thereof will be omitted to avoid excessive complexity in the description of the present specification.

### Advantageous Effects

Features and advantages of the present disclosure are summarized as follows.
(a) The present disclosure provides an information-providing method for diagnosing acute exacerbation of bronchiectasis.
(b) The present disclosure provides a diagnostic kit for diagnosing acute exacerbation of bronchiectasis.
(c) The use of the method and kit of the present disclosure allows for easy diagnosis of acute exacerbation of bronchiectasis through a quantitative indicator.

### Brief Description of the Drawings

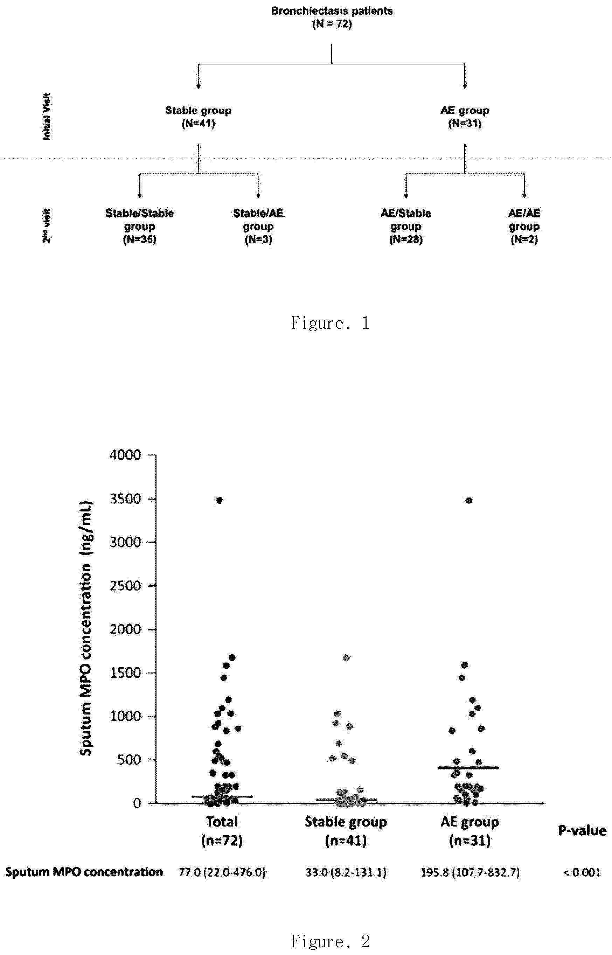

FIG. 1 shows the study population. The abbreviation AE represents acute exacerbation. Three patients in the stable group and one patient in the AE group at the initial visit did not visit the hospital or could not participate in the measurement due to a small volume of sputum at the second visit.
FIG. 2 shows the comparison results of the MPO concentration between the stable group and the AE group. The abbreviations AE and MPO represent acute exacerbation and myeloperoxidase, respectively.

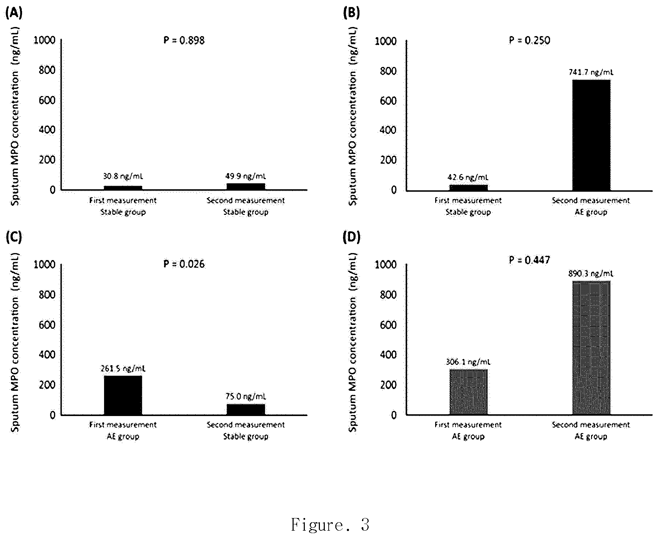

FIG. 3 shows the MPO concentration change according to the change in bronchiectasis state. (A) shows the MPO concentration change in the stable/stable group, (B) shows the MPO concentration change in the stable/AE group, (C) shows the MPO concentration change in the AE/stable group, and (D) shows the MPO concentration change in the AE/AE group. The patients were classified into four groups: stable/stable, stable/AE, AE/stable, and AE/AE, according to the disease state at the first and second visits. The abbreviations AE and MPO represent acute exacerbation and myeloperoxidase, respectively.

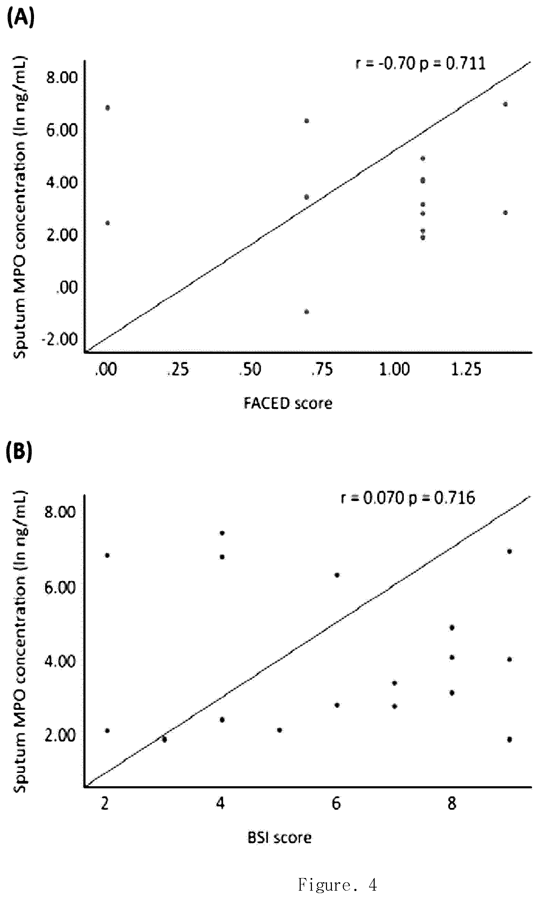

FIG. 4 shows the correlation between the severity of bronchiectasis and the MPO concentration of the stable group. The abbreviation MPO represents myeloperoxidase, BSI represents bronchiectasis severity index, and FACED represents forced expiratory volume in 1 sec (F), age (A), chronic colonization by *Pseudomonas aeruginosa* (C), radiological extension (number of affected lung lobes), and dyspnea (D).

### Mode for Carrying Out the Invention

Hereinafter, the present disclosure will be described in more detail with reference to exemplary embodiments. These exemplary embodiments are provided only for the purpose of illustrating the present disclosure in more detail, and therefore, according to the purpose of the present disclosure, it would be apparent to a person skilled in the art that these exemplary embodiments are not construed to limit the scope of the present disclosure.

### Examples

### Example 1: Subject identification

72 Bronchiectasis patients visiting the hospital were prospectively enrolled. All the participants received a sputum examination at the time of study enrollment. Typically, patients in a stable group and an acute exacerbation (AE) group were followed for 3 months and 1-3 weeks, respectively. At the second visit, the patients were again examined for their conditions and received sputum examination. As a result, the patients were classified into four groups depending on the disease states at the first and second visits (FIG. 1A). Among the patients belonging to the stable group at the first visit, patients showing a stable state and patients showing an AE state at the second visit were classified as a stable/stable group and a stable/AE group, respectively. Similarly, among the patients belonging to the AE group at the first visit, patients showing a stable state at the second visit and patients showing an AE state at the second visit were classified as an AE/stable group and an AE/AE group, respectively. The acute exacerbation of bronchiectasis is defined as three or more main symptoms lasting for at least 48 h, and therefore, a change of treatment is required (Hill AT, Haworth CS, Aliberti S, Barker A, Blasi F, Boersma W, Chalmers JD, De Soyza A, Dimakou K, Elborn JS et al. Pulmonary exacerbation in adults with bronchiectasis: a consensus definition for clinical research. The European respiratory journal 2017; 49). The clinical state and severity of bronchiectasis were assessed by using FACED (forced expiratory volume in 1 sec (FEV₁) %predicted, age, chronic colonization, extension, and dyspnea) and bronchiectasis severity index (BSI) scores as described above (Chalmers JD, Goeminne P, Aliberti S, McDonnell MJ, Lonni S, Davidson J, Poppelwell L, Salih W, Pesci A, Dupont LJ. The bronchiectasis severity index. An international derivation and validation study. Am J Respir Crit Care Med 2014; 189: 576-85). The sputum MPO function profile was measured using AnyLab F Myeloperoxydase (Z-Biotech, South Korea), which is used for a point of care testing (POCT) method based on immunofluorescence. The first study results were to assess whether there was a difference in sputum MPO concentration depending on the disease state in a bronchiectasis patient.

### Example 2: Analysis of correlation between acute exacerbation (AE) and MPO concentration

The mean age of the study population was 66 years (interquartile range [IQR], 59-72 years), and the patients were 40 males (55.6%) and 50 non-smokers (69.4%). The mean body mass index (BMI) was 21.3 kg/m² (IQR, 19.5-23.3 kg/m²). The most common comorbid diseases were COPD (33.3%), followed by tuberculosis (27.8%) and then non-tuberculous mycobacterial pulmonary disease (25.0%). Microorganisms were confirmed in 48.6% of patients, and *Pseudomonas aeruginosa* (23.6%) was the most common. The median corrected Reiff, FACED, and BSI scores were 8 (IQR, 5-13), 2 (IQR, 1-3), and 8 (IQR, 6-9), respectively. There were no significant differences between groups in terms of age, sex, BMI, smoking history, comorbid diseases, lung function, microbiology, modified Reiff score, BSI score, FACED score, and laboratory results.

However, the baseline MPO concentration was significantly higher in the AE group than in the stable group (median 195.8 ng/mL [107.7-832.7] vs. 33.0 ng/mL, [IQR 8.2-131.1], p<0.001) (FIG. 1B). In the stable/stable group, there was no significant change in MPO concentration between the first measurement and the second measurement (median 30.8 ng/mL [IQR 6.4-218.0] vs. 49.9 ng/mL [IQR 15.8-231.7], p=0.898). In contrast, in the stable/AE group, the second MPO concentration was increased compared with the first MPO concentration, with no statistical significance (median 741.7 ng/mL[IQR 108.4-1194.3] vs. 42.6 ng/mL[IQR 22.4-59.0], p=0.250) (FIG. 1C). In the AE/stable group, the second MPO concentration was significantly reduced compared with the first MPO concentration (median 261.5 ng/mL[IQR 127.7-1042.9] vs. 75.0 ng/mL[IQR 26.1-522.7], p=0.026). In contrast, in the AE/AE group, there was no significant change in MPO concentration (median 306.1 ng/mL [IQR 89.9-450.0] vs. 890.3 mg/dL [IQR 517.0-818.5], p=0.447) (FIG. 1C). As shown in FIG. 1D, there was no statistically significant correlation between either the BSI or FACED score and MPO in the stable group (p>0.05 for both indicators).

Through the above result analysis, the MPO concentration for determining acute exacerbation (AE) could be derived. The stable group showed an MPO concentration of 50 ng/mL or more. However, the group of patients recovering to a normal state among the patients diagnosed with acute exacerbation within 3 weeks prior to the measurement of the MPO concentration was observed to show a median value of 75 ng/mL.

The present disclosure reflected the results of the initial study investigating whether the change in sputum MPO concentration could reflect the disease state of bronchiectasis. The present inventors observed no significant correlation between the sputum MPO concentration measured by BSI and FACED scores and the disease severity. However, the sputum MPO concentration was a good indicator of a current disease state as well as a change in disease state in bronchiectasis patients, showing that the MPO concentration was a potential biomarker reflecting the disease state (acute exacerbation (AE) or non-exacerbation) of bronchiectasis. It was established in the present disclosure that the sputum MPO concentration was a biomarker reflecting the disease state (acute exacerbation or non-exacerbation) rather than the disease severity. The present inventors observed no correlation between the sputum MPO concentration and the disease severity, which was assessed using BSI and FACED scores. In contrast, the sputum MPO concentration had a significant correlation with the state of bronchiectasis. That is, the sputum MPO concentration can reflect the severity of neutrophilic inflammation that was correlated with the AE state of bronchiectasis. In addition, MPO can contribute to airway obstruction and an increase in sputum production in bronchiectasis, which may be associated with **AE.**

The present disclosure has two important clinical significance. First, the results of the present disclosure show that the sputum MPO concentration can be used as a POCT biomarker for assessing the AE state and recovery in a bronchiectasis patient since the sputum MPO concentration was measured using the POCT method. The MPO-guided assessment of AE of bronchiectasis can lead to a clinical decision with respect to antibiotic prescription, which can help reduce unnecessary antibiotic use. Second, MPO can be a target of new neutrophil regulation therapy for AE treatment in bronchiectasis patients. Recently, brensocatib, a new dipeptidyl peptidase 1 inhibitor, has been shown to reduce the AE of bronchiectasis associated with the regulation of neutrophil elastase 3. Similarly, MPO inhibitors may be used as novel drugs to reduce AE of bronchiectasis.

In conclusion, the MPO concentration significantly correlated with a change in disease state (acute exacerbation or non-exacerbation of bronchiectasis).

Although the present disclosure has been described in detail with reference to the specific features, it will be apparent to those skilled in the art that this description is only for a preferred embodiment and does not limit the scope of the present disclosure.

## Claims

1. An information providing method for diagnosing acute exacerbation of bronchiectasis, the method comprising measuring the myeloperoxidase (MPO) concentration in a biological sample isolated from a subject, wherein the subject is determined as being in an acute exacerbation state of bronchiectasis if the measured MPO concentration exceeds 50 ng/mL.

2. The method of claim 1, wherein the subject is a subject suffering from bronchiectasis.

3. The method of claim 1, wherein the subject is a subject without an acute exacerbation state within three weeks.

4. The method of claim 1, wherein the biological sample is sputum.

5. The method of claim 1, wherein the measuring of the myeloperoxidase (MPO) concentration is performed by the following steps:
(a) mixing the biological sample with a detection buffer comprising a first anti-MOP antibody labeled with a fluorescent element;
(b) allowing the mixture in step (a) to react with a membrane coated with a second anti-MPO antibody; and
(c) measuring the MPO concentration in the biological sample through fluorescent detection of the fluorescent element after step (b).

6. The method of claim 5, wherein before step (a), the following step is further performed:
(pre-a) diluting the biological sample with a dilution buffer.

7. The method of claim 6, wherein in step (pre-a), the biological sample is diluted with the dilution buffer at a volume ratio of 1:5(v/v) to 1:20(v/v).

8. The method of claim 5, wherein the fluorescent element is any one selected from the group consisting of Indocyanine green (ICG), IRDye 800CW carboxylate (IRDye 800CW), Alexa Fluor-355, Alexa Fluor 488, Alexa Fluor 532, Alexa Fluor 546, Alexa Fluor-555, Alexa Fluor 568, Alexa Fluor 594, Alexa Fluor 647, Alexa Fluor 660, Alexa Fluor 680, Alexa Fluor 700, Alexa Fluor 750, Alexa Fluor 780, Flamma 749, Flamma 774, Flamma 800, FSD FluorTM 647, FSD FluorTM 680, FSD FluorTM 750, FSD FluorTM 800, sulfo-cyanine 3.5 carboxylic acid (Cy3.5), sulfo-cyanine 5 carboxylic acid (Cy5), sulfo-cyanine 5.5 carboxylic acid (Cy5.5), sulfo-cyanine 7 carboxylic acid (Cy7) or sulfo-cyanine 7.5 carboxylic acid (Cy7.5), Texas Red, Pacific Blue, Oregon Green 488, JOE, Lissamine, Rhodamine Green, BODIPY, fluorescein isothiocyanate (FITC), carboxy-fluorescein (FAM), Allophycocyanin (APC), phycoerythrin (PE), rhodamine, dichlororhodamine (dRhodamine), carboxy tetramethylrhodamine (TAMRA), carboxy-X-rhodamine (ROX), PicoGreen, and RiboGreen.

9. A kit for diagnosing acute exacerbation of bronchiectasis, the kit comprising a membrane coated with an anti-MPO antibody or an antigen-binding fragment thereof, wherein the kit is provided in the form of a cartridge for immunofluorescence measurement.

10. The kit of claim 9, wherein the membrane is a nitrocellulose membrane.
